# EUROPEAN PATENT APPLICATION

(11) **EP 1 800 677 A1**
(43) Date of publication of application: **27.06.2007**
(21) Application number: 05790516.8
(22) Date of filing: 04.10.2005
(51) Int. Cl.: A61K 31/4045, A61P 13/02, A61P 13/08, C07D 209/14

(54) **PREVENTIVE AND/OR THERAPEUTIC AGENT FOR URINE COLLECTION DISORDER ACCOMPANYING LOWER URINARY TRACT OBSTRUCTION**

(30) Priority: 05.10.2004 JP 2004292731
(71) Applicant: Kissei Pharmaceutical Co., Ltd., Matsumoto-shi Nagano 399-8710 (JP)
(72) Inventor: OMORI, Yasuhiro, Kissei Pharmaceutical Co., Ltd., Tokyo 112-0002 (JP); ARAI, Nobuhiko, Kissei Pharmaceutical Co., Ltd., Tokyo 112-0002 (JP); SHIMIZU, Tomoji, Kissei Pharmaceutical Co., Ltd., Tokyo 112-0002 (JP); OKUBO, Yoshio, Kissei Pharmaceutical Co., Ltd., Tokyo 112-0002 (JP)
(74) Representative: Hayes, Adrian Chetwynd
(86) International application number: PCT/JP2005/018334
(87) International publication number: WO 2006/038611

(57) **Abstract**

An agent for the prevention and/or treatment of urine collection disorders associated with lower urinary tract obstructive disease, **characterized by** containing an indoline derivative represented by the following general formula (I): 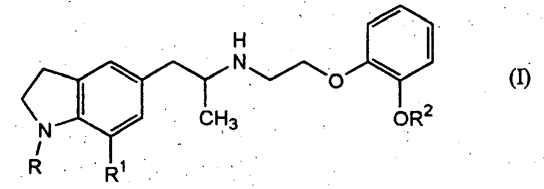 wherein R represents optionally substituted aliphatic acyl optionally having an unsaturated bond, hydroxyalkyl, aliphatic acyloxyalkyl, lower alkoxy, carboxy, lower alkoxycarbonyl, aryl(lower alkoxy) carbonyl, carbamoyl, (mono- or,dialkyl)carbonyl or cyano(lower alkyl), optionally halogenated aromatic acyl, furoyl, or pyridylcarbonyl; R¹ represents cyano or carbamoyl; and R² represents lower alkyl optionally having a halogen atom, cyano group, or aryl group, or a pharmaceutically acceptable salt of the derivative. The derivative and salt are usable as an agent for the prevention and/or treatment of urine collection disorders associated with lower urinary tract obstructive disease.

## Description

### Technical Field

The present invention relates to an agent for the prevention and/or treatment of storage disorder associated with lower urinary tract obstructive disease. More specifically, it relates to an agent for the prevention and/or treatment of storage disorder associated with lower urinary tract obstructive disease, which comprises an indoline derivative or a pharmaceutically acceptable salt thereof.

### Background Art

Lower urinary tract obstructive disease is a disease in which the lower urinary tract is obstructed by a prostatic disorder, a urethral disorder or the like, and the symptoms shown by urinary disturbance associated therewith include an obstructive symptom (voiding disorder) and irritative symptom (storage disorder). Examples of the obstructive symptom include difficulty of urination (e.g., delay of the start of micturition, prolonged micturition time, terminal dribbling, forceless thin stream, two-phase micturition, intermittent micturition and the like) and urinary retention. In addition, examples of the irritative symptom include frequent micturition, nocturia, urgency, urinary incontinence and the like (cf. Non-patent Reference 1).

In general, an α₁-adrenoceptor (referred sometimes to as "AR" hereinafter) blocker and a cholinergic drug, which have a urinary tract smooth muscle relaxing action, are used for the voiding disorder, and an anti-cholinergic drug, a tricyclic antidepressant, a β,α-AR stimulant and the like, which have the action to inhibit over-contraction of diuretic muscle and thereby increase the bladder capacity, are used for the storage disorder (cf. Non-patent Reference 2).

It is known that, among α₁-AR subtypes, an α_{1D}-AR subtype blocker is effective for diuretic muscle contraction which causes storage disorder (cf. Patent Reference 1 and Non-patent Reference 3). This is proved also by a study on α₁-AR subtypes carried out by measuring mRNA distribution, reporting that while α_{1A} is predominantly expressed in the prostate gland which is concerned in the voiding disorder, α_{1D} is predominantly expressed in the bladder and spinal cord which are concerned in the storage disorder (cf. Non-patent Reference 4).

An indoline derivative represented by the following general formula (I) or a pharmaceutically acceptable salt thereof is a markedly useful compound as a therapeutic agent for urinary disturbance associated with benign prostatic hyperplasia and the like, because it has a selective action to suppress contraction of urinary tract smooth muscle and can lower urethral pressure without greatly exerting influence upon blood pressure (cf. Patent Reference 2).

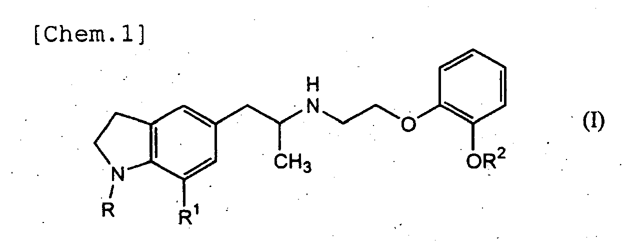

In the formula, R represents an aliphatic acyl group which may have one or more of halogen atom, hydroxyl group, a lower alkoxy group, a carboxyl group, a lower alkoxycarbonyl group, a cycloalkyl group or an aryl group as its substituent group and may have an unsaturated bond in some cases, a hydroxyalkyl group, an aliphatic acyloxyalkyl group, a lower alkyl group which has a lower alkoxy group, carboxy group, a lower alkoxycarbonyl group, an aryl-substituted lower alkoxycarbonyl group, a carbamoyl group, a mono- or dialkyl-substituted carbonyl group or a cyano group as a substituent group, an aromatic acyl group which may have one or more of halogen atom as a substituent group, furoyl group or pyridylcarbonyl group, R¹ represents a cyano group or a carbamoyl group, and R² represents a lower alkyl group which may have one or more of a halogen atom, a cyano group or an aryl group as its substituent group.

Among the aforementioned indoline derivatives, a compound named KMD-3213 (general name: silodosin) selectively acts upon α_{1A}-AR subtype, but hardly acts upon α_{1B} and α_{1D}-AR subtypes, and what is more, it does not show inverse agonist activity, so that it is markedly excellent as a therapeutic agent for the dysuria associated with prostatic hyperplasia (cf. Patent Reference 3 and Non-patent Reference 5).

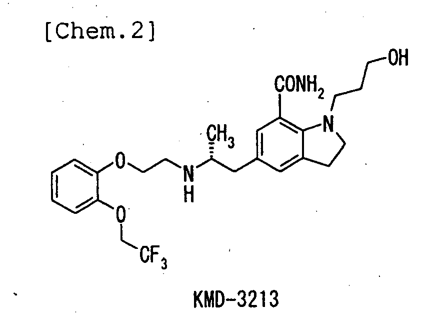

Recently, it has been proposed that certain urinary disturbance symptoms which are associated with functional obstruction of lower urinary tract, but excluding those which are caused by a disturbance of the nerve that controls the lower urinary tract or by an organic disturbance of the lower urinary tract, should be newly classified as a disease called lower urinary tract disease, and it has been reported that silodosin is effective for this (cf. Patent Reference 4). However, the Patent Reference 4 does not describe that silodosin is effective for a storage disorder associated with an organic disorder as the obstruction of the lower urinary tract, or there is no description suggesting the same.

On the other hand, it has been drawing attention to a morbid state called overactive bladder which is defined as a medical condition that causes frequent micturition and urgency regardless of the presence or absence of stress incontinence, and that has no topical pathological condition or metabolic factor possibly causing such a symptom. It has been reported that tamsulosin having α_{1A}-AR blocking action and α_{1D}-AR blocking action is effective for this overactive bladder (cf. Patent Reference 5). However, Patent Reference 5 does not describe that indoline derivatives such as silodosin or pharmaceutically acceptable salts thereof are effective for storage disorders such as frequent micturition, urgency and the like. In addition, it has been considered that not a drug showing an α_{1A}-AR blocking action regarding obstructive symptom (voiding disorder), but a drug showing an α_{1D}-AR blocking action regarding irritative symptom (storage disorder) is effective for the overactive bladder (cf. Non-patent Reference 6). Thus, those skilled in the art cannot easily think of the application of indoline derivatives such as silodosin or pharmaceutically acceptable salts thereof, which are α_{1A}-AR-selective blocking agents hardly showing α_{1D}-AR blocking action, to the prevention and/or treatment of storage disorders.
Patent Reference 1: International Publication No. 99/57131
Patent Reference 2: JP-A-6-220015
Patent Reference 3: JP-A-2000-247998
Patent Reference 4: JP-A-2001-288115
Patent Reference 5: International Publication No. 03/103659
Non-patent Reference 1: Kosaku Yasuda et al., "Hainyo Shogai no Yakubutsu Chiryo (Drug Therapy of Urinary Disturbance)", Miwa Shoten, 2000, pp.36-43
Non-patent Reference 2: Satoru Takahashi, Jin to Toseki (Kidney and Dialysis), 2002, Special Issue, pp.99-101
Non-patent Reference 3: Baojun Gu et al., The Journal of Urology, American Urological Association, 2004, vol. 172, pp.758-762
Non-patent Reference 4: Keiichi Shishido et. al., Rinsho Hinyoki Ka (Clinical Urology), 2003 Special Issue, vol. 57, no. 4, pp.104-108
Non-patent Reference 5: Yamagishi et al., European Journal of Pharmacology, 1996, no. 315, pp.73-79 Non-patent Reference 6: Donna J. Sellers et al., World J. Urol., 2001, vol. 19, p.308

### Disclosure of the Invention

### Problems that the Invention is to Solve

The present invention aims at providing an agent for the prevention and/or treatment of storage disorder associated with lower urinary tract obstructive disease.

### Means for Solving the Problems

Taking the aforementioned problems into consideration, the present inventors have conducted intensive studies and found to our surprise that an indoline derivative represented by the aforementioned general formula (I) or a pharmaceutically acceptable salt thereof, which, being an α_{1A}-AR-selective blocker that hardly shows α_{1D}-AR blocking action, has been considered to be not applicable to a storage disorder in which diuretic muscle contraction is concerned, is markedly effective for a storage disorder associated with lower urinary tract obstructive disease, thus accomplishing the present invention.

That is, the gist of the present invention resides in an agent for the prevention and/or treatment of a storage disorder associated with lower urinary tract obstructive disease, which comprises an indoline derivative represented by the general formula (I) or a pharmaceutically acceptable salt thereof.

### Effect of the Invention

The agent for the prevention and/or treatment of a storage disorder associated with lower urinary tract obstructive disease, which comprises an indoline derivative represented by the general formula (I) or a pharmaceutically acceptable salt thereof shows a remarkable therapeutic effect for patients having storage disorders.

### Best Mode for Carrying Out the Invention

In the general formula (I), the term "lower alkyl" means a straight chain or branched chain alkyl having 1 to 6 carbon atoms, the term "hydroxyalkyl" means a straight chain or branched chain alkyl having 2 to 6 carbon atoms and having a hydroxyl group, wherein said hydroxyl group is present at a position other than the α-position, the term "lower alkoxy" means a straight chain or branched chain alkoxy having 1 to 6 carbon atoms, and the term "cycloalkyl" means a 5- to 7-membered cyclic alkyl, respectively. Also, the term "aryl" means an aromatic hydrocarbon such as a phenyl, naphthyl or the like, the term "aromatic acyl" means an acyl of a carboxylic acid having an aryl which has the same meaning as the above, the term "aliphatic acyl which may have an unsaturated bond" means an acyl of a straight chain or branched chain alkylcarboxylic acid having 2 to 7 carbon atoms or a straight chain or branched chain alkenylcarboxylic acid having 3 to 7 carbon atoms, and the term "aliphatic acyloxy" means an alkylcarbonyloxyalkyl having 4 to 13 carbon atoms and having a hydroxyl group substituted with the aforementioned aliphatic acyl group, wherein said aliphatic acyloxy group is present at a position other than the α-position, respectively. In addition, the term "furoyl" means 2-furoyl or 3-furoyl, the term "pyridylcarbonyl" means 2-pyridylcarbonyl, 3-pyridylcarbonyl or 4-pyridylcarbonyl, and the term "halogen atom" means a fluorine atom, a chlorine atom or a bromine atom, respectively. In this connection, the indoline derivative of general formula (I) can be prepared by the method described in Patent Reference 2, and as the indoline derivatives, the aforementioned silodosin, namely (-)-1-(3-hydroxypropyl)-5-((2R)-2-{[2-({2-[(2,2,2-trifluoroethyl)-oxy]phenyl}oxy)ethyl]amino}propyl)2,3-dihydro-1H-indole-7-carboxamide, is preferable.

As the pharmaceutically acceptable salt of the aforementioned indoline derivative, for example; a compound having a carboxy group may be converted into its salt with an inorganic base such as sodium, potassium, calcium or the like or with an organic amine such as morpholine, piperidine or the like. Also, among the indoline derivatives, a compound in which the substituent group R is a substituted or unsubstituted acyl group or furoyl group may be converted into its monoacid addition salt with hydrochloric acid, hydrobromic acid, sulfuric acid, methanesulfonic acid, benzenesulfonic acid, p-toluene-sulfonic acid, acetic acid, citric acid, succinic acid, tartaric acid, 2,4-dimethylbenzenesulfonic acid, 2,4,6-trimethylbenzenesulfonic acid, (+)-camphorsulfonic acid, (-)-camphorsulfonic acid, 4-chlorobenzenesulfonic acid, 2-naphthalenesulfonic acid, 1-butanesulfonic acid, fumaric acid, glutamic acid, aspartic acid or the like. In addition, among the indoline derivatives, a compound in which the substituent group R is a substituted alkyl group or pyridylcarbonyl group may be converted into its monoacid addition salt with hydrochloric acid, hydrobromic acid, sulfuric acid, methanesulfonic acid, p-toluenesulfonic acid, 2,4-dimethylbenzenesulfonic acid, 2,5-dimethylbenzenesulfonic acid, 2,4,6-trimethylbenzenesulfonic acid, (+)-camphorsulfonic acid, (-)-camphorsulfonic acid, 4-chlorobenzenesulfonic acid, 2-naphthalenesulfonic acid, 1-butanesulfonic acid, fumaric acid, glutamic acid, aspartic acid or the like.

Since the indoline derivative represented by general formula (I) or a pharmaceutically acceptable salt thereof shows markedly high selectivity for α_{1A}-AR which is concerned in the contraction of human prostate gland, it is preferable that the lower urinary tract obstructive disease is benign prostatic hyperplasia.

The agent for the prevention and/or treatment of a storage disorder associated with lower urinary tract obstructive disease of the present invention can be prepared by mixing the aforementioned indoline derivative represented by general formula (I) or a pharmaceutically acceptable salt thereof with commonly used drug preparation carriers.

The drug preparation carriers may be used by optionally combining them in response to each administration form, and their examples include excipients such as lactose and the like; lubricants such as magnesium stearate and the like; disintegrating agents such as carboxymethylcellulose and the like; binders such as hydroxypropylmethylcellulose and the like; surfactants such as macrogol and the like; foaming agents such as sodium bicarbonate and the like; solubilizing agents such as cyclodextrin and the like; acidity agents such as citric acid and the like; stabilizing agents such as sodium edetate and the like; pH adjusting agents such as phosphate and the like, and the like.

Examples of the administration form of the agent for the prevention and/or treatment of a storage disorder associated with lower urinary tract obstructive disease of the present invention include, for example, oral administration preparations such as powders, granules, fine subtilaes, dry syrups, tablets, capsules and the like; parenteral administration preparations such as injections, patches, suppositories and the like, and the like, of which oral administration preparations are preferable.

It is preferable to prepare the aforementioned preparations in such a manner that the indoline derivative represented by general formula (I) or a pharmaceutically acceptable salt thereof is administered within the range of from 2 to 16 mg, particularly from 4 to 8 mg, per day per adult, as the oral administration preparations.

The agent for the prevention and/or treatment of a storage disorder associated with lower urinary tract obstructive disease of the present invention may further contain other drug for a storage disorder, preferably a drug for a storage disorder having different action mechanism. Examples of such a drug for a storage disorder having different action mechanism include muscarine receptor antagonists such as oxybutynin, tolterodine, darifenacin, nuvenzepine, zamifenacin, tiotropium, albamelin, trospium, fesoterodine, temebeline, quinuclidin-3-yl 1-phenyl-1,2,3,4-tetrahydroisoquinoline-2-carboxylate, 4-(2-methyl-1H-imidazolyl-1-yl)-2,2-diphenylbutylamide and N-[1-(6-aminopyridin-2-ylmethyl)piperidin-4-yl]-2(R)-[3,3-difluoro-1(R)-cyclopentyl]-2-hydroxy-2-phenylacetamide and the like; and the like.

### Examples

The following describes the present invention further in detail based on Examples, but the present invention is not limited to the contents.

### [Example 1] Clinical effects on the storage disorders of urinary disturbance associated with prostatic hyperplasia

Using patients of urinary disturbance associated with benign prostatic hyperplasia as the objects, clinical effects of silodosin and tamsulosin hydrochloride were examined by a placebo-control parallel groups-comparing double blind trial, using changes in the total I-PSS (International Prostate Symptom Score) from pre-administration as the primary endpoint. The I-PSS consists of 3 items of irritative symptoms representing the symptoms at the time of storing (Nocturia, Urgency and Urination within 2 hours) and 4 items of obstructive symptoms representing the symptoms at the time of voiding (Sensation of residual urine, Intermittency of urinary stream, Power of urinary stream and Straining during urination), prepared fo use in the judgment of various therapeutic effects on the dysuria associated with benign prostatic hyperplasia, but this is not specific to prostatic hyperplasia and can also be used for the judgment of therapeutic effects on female dysuria and the like (Non-patent Reference 1).

Effects of silodosin (4 mg per once, twice a day) and tamsulosin hydrochloride (0.2 mg per once, once a day) on the storage disorder were examined by the following method.
Patients to be tested: 455 patients of dysuria associated with benign prostatic hyperplasia
Administration method: oral administration for 12 weeks
Primary endpoints: total score of I-PSS, I-PSS irritative symptom score and I-PSS obstructive symptom score
Analysis method: all of the finally evaluated cases were stratified into groups of patients having a total score of less than 6 and that of 6 or more regarding the "Urination within 2 hours" and "Urgency" of I-PSS, and average values of respective changes in the total score of I-PSS, I-PSS irritative symptom score and I-PSS obstructive symptom score before and after the administration were calculated for each group.
Administration groups: silodosin group (116 cases having a pre-administration score of less than 6, and 58 cases having that of 6 or more), tamsulosin group (135 cases having a pre-administration score of less than 6, and 57 cases having that of 6 or more), a placebo group (61 cases having a pre-administration score of less than 6, and 28 cases having that of 6 or more)

### I-PSS items questioned

(1) Sensation of residual urine
   "Have you had a sensation of not emptying your bladder completely after you finished urinating?"
(2) Urination within 2 hours
   "Have you had to urinate again less than two hours after you finished urinating?"
(3) Intermittency of urinary stream
   "Have you found you stopped and started again several times when you urinated?"
(4) Urgency
   "Have you found it difficult to postpone urination?"
(5) Power of urinary stream
   "Have you had a weak urinary stream?"
(6) Straining during urination
   "Have you had to push or strain to begin urination?"
   The scores of. (1) to (6) are as follows.
   Not at all: 0 point; Less than 1 time in 5: 1 point; Less than 1 time in 2: 2 points; About 1 time in 2: 3 points; More than 1 time in 2: 4 points; Almost always: 5 points.
(7) Nocturia
   "How many times did you get up to urinate from the time you went to bet at night until the time you got up in the morning?"
   The scores are as follows.
   None: 0 point; 1 time: 1 point; 2 times: 2 points; 3 times: 3 points; 4 times: 4 points; 5 times or more: 5 points.

Results are shown in Table 1.

**[Table 1]**

| Table 1. Scores of I-PSS by each symptom classified before and after 6 of the total score at pre-administration "Urination within 2 hours" and "Urgency" | | | | | | | |
|---|---|---|---|---|---|---|---|
| I-PSS (Pre-admin*) | Groups | Total I-PSS | | I-PSS. (irritative symptom) | | I-PSS (obstructive symptom) | |
| | | Pre-admin.* | Changes | Pre admin.* | Changes | Pre admin.* | Changes |
| Less than 6 | Silodosin | 14.7 | -6.8 | 4.9 | -1.6 | 9.9 | -5.2 |
| | Tamsulosin | 14.8 | -5.9 | 4.8 | -1.3 | 10.0 | -4.6 |
| | Placebo | 14.4 | -4.1 | 4.9 | -0.9 | 9.6 | -3.2 |
| 6 or more | Silodosin | 21.9 | -11.3 | 9.6 | -4.3 | 12.5 | -7.1 |
| | Tamsulosin | 22.2 | -9.1 | 9.6 | -3.9 | 12.6 | -5.2 |
| | Placebo | 22.9 | -7.9 | 9.2 | -2.8 | 13.7 | -5.1 |
| Overall | Silodosin | 17.1 | -8.3 | 6.4 | -2.5 | 10.8- | -5.8 |
| | Tamsulosin | 17.0 | -6.8 | 6.2 | -2.1 | 10.8 | -4.8 |
| | Placebo | 17.1 | -5.3 | 6.3 | -1.5 | 10.9 | -3.8 |

| | | | | | | | |
|---|---|---|---|---|---|---|---|
| *: Pre-administration | | | | | | | |

It can be seen from Table 1 that silodosin has the effect to improve I-PSS irritative symptom score in patients showing storage disorders, particularly patients having a 6 or more total score of the "Urination within 2 hours" and "Urgency" of I-PSS at pre-administration, and therefore is effective as an agent for the prevention and/or treatment of storage disorders.

## Claims

1. An agent for the prevention and/or treatment of a storage disorder associated with a lower urinary tract obstructive disease, which comprises an indoline derivative represented by a general formula (I): 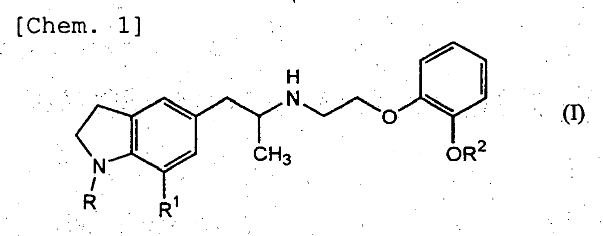 in the formula, R represents an aliphatic acyl group which may have one or more of halogen atom, hydroxyl group, lower alkoxy group, carboxy group, lower alkoxycarbonyl group, cycloalkyl group or aryl group as its substituent group and may have an unsaturated bond in some cases, a hydroxyalkyl group, an aliphatic acyloxyalkyl group, a lower alkyl group which has a lower alkoxy group, a carboxy group, a lower alkoxycarbonyl group, an aryl-substituted lower alkoxycarbonyl group, a carbamoyl group, a mono- or dialkyl-substituted carbonyl group or cyano group as a substituent group, an aromatic acyl group which may have one or more of halogen atom as a substituent group, a furoyl group or a pyridylcarbonyl group, R¹ represents a cyano group or a carbamoyl group, and R² represents a lower alkyl group which may have one or more of halogen atom, cyano group or aryl group as a substituent group, or a pharmaceutically acceptable salt thereof.

2. The agent for the prevention and/or treatment of a storage disorder associated with a lower urinary tract obstructive disease as claimed in claim 1, wherein the indoline derivative is silodosin.

3. The agent for the prevention and/or treatment of a storage disorder associated with a lower urinary tract obstructive disease as claimed in claim 1 or 2, wherein the lower urinary tract obstructive disease is benign prostatic hyperplasia.

4. The agent for the prevention and/or treatment of a storage disorder associated with a lower urinary tract obstructive disease as claimed in any one of claims 1 to 3, which is administered to a patient having a total score of 6 or more as the "Urination within 2 hours" and "Urgency" before administration by the international prostate symptom score.

5. The agent for the prevention and/or treatment of a storage disorder associated with a lower urinary tract obstructive disease as claimed in any one of claims 1 to 4, wherein daily dose of the indoline derivative represented by general formula (I) or a pharmaceutically acceptable salt thereof is from 2 to 16 mg.

6. The agent for the prevention and/or treatment of a storage disorder associated with a lower urinary tract obstructive disease as claimed in any one of claims 1 to 5, which further comprises other drug that may be used for a storage disorder.
